(19) Europäisches Patentamt
European Patent Office
Office européen des brevets

(11) Publication number: **0 117 321 B1**

(12) # EUROPEAN PATENT SPECIFICATION

(45) Date of publication of patent specification: **24.07.91**

(51) Int. Cl.5: **A23L 1/30**, A23C 9/158, A23G 3/30, //A23G9/02, A23C15/12,A23C19/076, A23C9/13

(21) Application number: **83201864.2**

(22) Date of filing: **29.12.83**

The file contains technical information submitted after the application was filed and not included in this specification

(54) **Foodstuffs, including chewing gum with anti-caries activity, and processes for preparing them.**

(30) Priority: **29.12.82 NL 8205030**

(43) Date of publication of application:
**05.09.84 Bulletin 84/36**

(45) Publication of the grant of the patent:
**24.07.91 Bulletin 91/30**

(84) Designated Contracting States:
**AT BE CH DE FR GB IT LI LU NL SE**

(56) References cited:
**CH-A- 397 401**
**DE-A- 2 621 215**
**FR-A- 2 516 355**
**US-A- 3 256 095**
**DE-A- 1 956 016**
**FR-A- 2 239 205**
**US-A- 3 219 454**

**Journal of food Science, vol. 47, n 1, January-February 1982, pages 228-230; Chicago, Illinois, US R.A. CLEMENS "Effects of storage on the bioavailability and chemistry of iron powders in a heat-processed liquid milk-based product". Page 228, column 2.**

(73) Proprietor: **TABAGH AG**
**CH-3984 Fiesch(CH)**

(72) Inventor: **Laimböck, Johannes Franciscus**
**Hofzichtlaan 1**
**NL-2594 CB The Hague(NL)**

(74) Representative: **Van Assen, Jan Willem Bernard**
**Octrooibureau van Assen Assenpatent B.V.**
**Konijnenlaan 22**
**NL-2243 ER Wassenaar(NL)**

EP 0 117 321 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

Netherlands Milk and Dairy Journal, vol. 29, no.1, January 1975, pages 3-15; Ede, NL. R.H.C. Elgersma et al. "The effect of fluoridated tap-water, used in the cheesemaking process, on the fluoride content of Gouda cheese." Pages 3, 4, 8, 11 - 13.

Chemical abstracts, vol.49, July 10, 1955, column 9143i; Colombus, Ohio, US. R.S. Manly "Dental caries and dentrifices". Drug and Cosmetic Ind. 76, 326-7, 422-5 (1955).

Dictionnaire Vidal, 1981, page 1386, Office Vulg. Pharmac., Paris, FR. *Left-hand column "Vitamine K1 'Roche'" - right-hand column*.

Microbiologial Reviews, March 1978, pp. 45-66.

Reviews of infectious diseases, vol. 6 Nov./Dec. 1981, pp. 1127-1138.

Research report from Jwe Van Dijk Utrecht, Oct. 1987.

J. Periodontal 56, 1985, pp. 22-27.

## Description

The invention relates to foodstuff, including chewing-gum, with anti-caries activity, and processes for preparing them.

From the document US-A-3,219,454 is known a liquid low calorie diet containing vitamin K and iron and fluorine compounds (see column 4, table 4).

The said document US-A-3,219,454 refers principally to a liquid food containing several water soluble carbohydrates of varying rates of assimilation in the body, so that their energy is released over a widened period of time as shown by varying rates of intestinal absorption of the different carbohydrates.

The said diet contains lactose, sucrose, water soluble dextrins, dextrose and maltose in proportions in the order named, with lactose being the largest amount. The high proportion of lactose is considered to promote the development in the alimentary canal of flora which offset in part at least the shortage of bulking ingredients found in usual diets of this kind.

Besides the components stated, the food contains the usual types of flavoring materials, mineral and vitamin supplements, and for best results guar gum and carageen, either alone or mixed, or other viscosity increasing or thickening agents commonly used in foodproducts.

Applicant has found that foodstuff containing added vitamin K fluorine and iron compounds under fullfillment of a few other conditions are effective in checking caries.

The other conditions are that the foodstuff :

a) contains vitamin K in an amount effective for the reduction of caries;

b) contains one or more iron compounds in an amount from 1 to 1000 mg per 100 g of dry matter, assisting the caries resisting effect of the vitamin K ;

c) contains fluorine compounds, which provide from 0.001 to 0.75 mg of available fluorine per 100 g of dry matter ;

d) the iron and vitamin K are wholly or partly incorporated in a component of the foodstuff which component possesses the adherence to the teeth of cheese.

Applicant has discovered that the presence of iron in combination with vitamin K and fluorine has an anti-caries effect.

This anti-caries effect is surprising as one should expect from literature data that iron should just have a caries promoting effect.

According to the publications "Iron and Infection" by Eugene D. Weinberg in Microbiological Reviews, March 1978, p. 45-66 and "The Significance of Iron in Infection" by J.J. Bullen in Reviews of Infection Diseases, vol. 3., no. 6, November-December 1981, p. 1127-1138, iron compounds are a nutrient for bacteria and for acid producing bacteria as well.

For this reason iron in body fluids is held less available to bacteria by means of iron-binding proteins and properties of the iron-binding and iron-transport systems of the host.

It is assumed by applicant that vitamin K has both an external activity, i.e. in the oral cavity by contact with the teeth and the gums and an internal activity, i.e. via the blood circulation. The external activity is a tanning and bacteriostatic activity, acting against gingivitis (inflammation of the gums, starting with inflammation of the skin of the gums). (It is known that quinone compounds generally have a powerful tanning activity). The reduction of gingivitis is important because gingivitis leads to the development of caries at the border of the gums and the tooth. The internal activity consists of increasing the healing ability of the gums, on the one hand, and according to recent views vitamin K plays a role in the calcium transport in the body and the growth of bones and teeth, on the other hand. As in the remineralisation of teeth both calcium and fluorine are involved, in a preferred embodiment the present invention provides a foodstuff to which besides one or more fluorine compounds, which provide from 0.001 to 0.75 mg of available fluorine per 100 g dry matter, also at least one vitamin K has been added, in an amount effective for the reduction of caries.

As there is evidence that the iron and vitamin K have a synergetic action on each other, according to the invention these components are wholly or partly incorporated in a component of the foodstuff which possesses the adherence to the teeth properties of cheese.

By "vitamin K" in the present application not only natural vitamins K (vitamin $K_1$ and $K_2$) are meant, but also synthetic vitamins K and compounds which are chemically closely related therewith, as far as they are comprised within the group of compounds of the formulae :

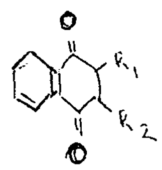
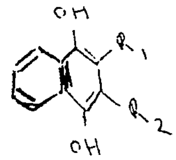
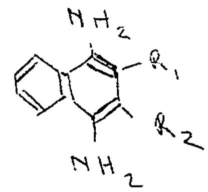

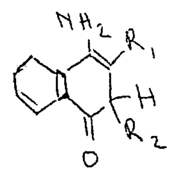
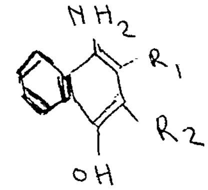

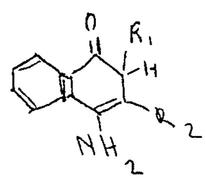
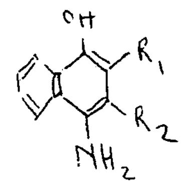

in which $R_1$ is ON or $CH_3$ and $R_2$ is H, OH, alkyl, hydroxy alkyl, alkenyl or hydroxy alkenyl with from 1 to 30 carbonatoms, and the oxides (particularly the 2,3-oxides and the physiologically acceptable esters, ethers and salts thereof, and similar compounds substituted in the left hand bezene nucleus. Examples include :

2-methyl-3-phytyl-1,4-naphthoquinone (vitamin $K_1$),
2-methyl-3-farnesyl-1,4-naphthoquinone (vitamin $K_2$),
2-methyl-1,4-naphthoquinone (vitamin $K_3$),
2-methyl-1,4-naphthoquinone-sodium bisulphite,
2-methyl-1,4-naphthohydroquinone,
2-methyl-1,4-naphthohydroquinone-1,4diacetate,
2-methyl-1,4-naphthohydroquinone-1,4-dipropionate,
2-methyl-1,4-naphthohydroquinone-1,4-dibutyrate,
2-methyl-1,4-naphthohydroquinone-1,4-diisobutyrate,
2-methyl-1,4-naphthohydroquinone-1,4-diphosphoric acid ester disodium salt,
2-methyl-1-oxy-4-amino-naphthalene,
2-methyl-1,4-diamino-naphthalene, and
2-methyl-1-amino-4-oxy-naphthalene.

A preferred foodstuff of the invention is characterized in that it comprises milk or a product derived from milk or from one or more milk constituents as a basis.

Whereas with many of the usual foodstuff, particularly those containing sugars, it is undesirable that remnants of it remain in contact with the teeth, remnants of the foodstuff of the invention on the contrary have a protecting and healing effect on the teeth and the gums. This effect will be even more pronounced as the foodstuff is more viscous, because in that case it will have a better adherence to the teeth. Therefore according to the invention a foodstuff that comprises a product derived from milk or from one or more milk constituents having a higher viscosity than milk as a basis, is preferred.

Although milk to which according to the invention a vitamin K and optionally a fluorine compound has been added can provide a contribution to the protection of the teeth against caries, this embodiment is not preferred, because milk contains much lactose, which in the oral cavity may be converted into lactic acid, by which the pH of the coating layer of the teeth is decreased and the risk of affection of the teeth is increased.

In this respect milk powder cannot be bracketed with milk ; in the first place because in the preparation thereof the lactose content is often decreased (by caramellisation by contact with hot air) ; in the second place because for a very substantial part milk powder is used for the production of yoghurt and other milk products.

The milk and products derived from milk, constituting the basis for the foodstuff of the present invention

have in common that they contain calcium and phosphate which are considered as important for preservation of a healthy set of teeth, because they can lead to remineralisation, the more so when, according to the present invention, a vitamin K and preferably also a fluorine compound is present in association therewith.

Vitamin $K_1$ is preferred, because of its good activity, associated with its very low toxicity : Hans Vogel, Chemie und Technik der Vitamine, Band I, page 331 (Ferdinand Cuhe Verlag, Stuttgart, 1950) mentions that in mice an amount of 25 g/kg body weight was not lethal. For 2-methyl-1,4-naphthoquinone and 2-methyl-1,4-naphthohydroquinone the lethal doses are about 0.6 and 0.3 g/kg, respectively. The 2-methyl-1,4-naphthohydroquinone-1,4-diesters show lethal doses of from 9 g/kg up to more than 18 g/kg.

The most preferred embodiment of the invention is the embodiment in which the foodstuff comprises cheese as a basis. Cheese has a very good adherence to the teeth. Moreover in its own right it already has favourable properties as a foodstuff in relation to the caries problem, as is known from the article by W.M. Edgar et al, "Effects of Different Eating Patterns on Dental Caries in the Rat" in Caries Res. 16, 384-389 (1982) from which it appears that caries in rats is reduced if the sucrose-rich diet that is provided in 24 portions per day is supplemented with cheese 12 times per day. The absence of lactose in cheese will be an important factor in this context.

In the present specification and claims the term "cheese" comprises not only cheese as defined in "Kaasbesluit (Warenwet)", 1981, Stb. 227 (Cheese Decree (Foods and Drugs Act) 1981, Statutebook 227), but also cottage cheese, and products similar to cheese and cottage cheese in which some of the constituents, e.g. the rennet, or parts of the milk fat and/or the milk protein, have been replaced by others. Also cheese obtaining starting from vegetable fats and/or proteins is considered.

Cheese in which according to the present invention vitamin K, and preferably also fluorine, has been included could play an important role in the fight against caries in school children. It could be considered to make available a daily portion of the cheese of the present invention, in nutritional value approximately equivalent to the school milk now current, but more easily distributable and moreover effective as a means for fighting caries, a disease which despite the great efforts of the past years still occurs on a large scale and which does not seem to be fightable in a conclusive way by the means currently used.

Other products which can serve as a basis for the foodstuff of the present invention are e.g. skimmed milk, partially skimmed milk, cocoa milk, buttermilk, cottage cheese, yoghurt, kefir, cream, custard, pudding, porridge, butter, butter oil, milk powders, condensed milk, ice-cream, and special food for babies and young children, etc.

Also products which are only partially based upon milk or upon one or more milk constituents are very suitable as a basis for the foodstuff of the present invention. Examples of such products are margarine, ice-cream, food for babies, young children, mothers, geriatric food, medical food, milk chocolate, stuffed chocolate, etc.

Foodstuff comprising a core and an envelope, in which only the core or only the envelope contains vitamin K and optionally fluorine are also suitable. In this context e.g. snacks with a core of a vitamin K and optionally fluorine containing substance and a chocolate envelope may be considered. Another example is cheese, containing one or more vitamins K and optionally one or more fluorine compounds and en-robed with a protecting layer of another edible material, e.g. gelatin.

Examples of foodstuff, not based upon milk or milk products, to which according to the invention vitamin K and preferably also a fluorine compound can be added, are bread, cake, marmelade, etc.

The amount of fluorine compound incorporated in the foodstuff of the invention is preferably from 0.01 to 0.75 mg, more preferably from 0.1 to 0.4 mg, calculated as available fluorine, per 100 g dry matter.

Suitable fluorine containing compounds comprise NaF, $CaF_2$, $Na_2PO_3F$, $SrF_2$, $NH_4PF_6$, $NH_4PO_2F_2$, $NH_4F$, KF, $NaPF_6$, $KPF_6$, bis-(hydroxy-ethyl)-aminopropyl-octadecylamine-dihydrofluoride and hexadecyclaminehydrofluoride. Preferred are sodium fluoride (NaF) and sodium fluorophosphate ($Na_2PO_3F$) or a combination of these compounds. For low-sodium-diets instead of sodium compounds the corresponding potassium or ammonium compounds are used.

The amount of vitamin K will vary with the potency of the particular compound, but generally it will be preferred to range between 0.001 and 100 mg, most preferably between 0.1 and 10 mg, per 100 g of dry matter. According to Vitamin-Compendium 1970, page 83 (F. Hoffmann-La Roche & Co., Basel, Switzerland) the daily vitamin K need for children is about 2 mg, and for adults about 4 mg, and according to Hans Vogel, Chemie und Technik der Vitamine, page 331 (cited above) higher doses are not toxious, and according to the present invention are even preferred. With a daily food-intake of 800 g of dry matter the ranges of from 0.001 to 100 mg and from 0.1 to 10 mg per 100 g of dry matter, respectively, correspond with a daily dose of from 0.008 to 800 mg and from 0.8 to 80 mg of vitamin K, respectively.

In order to prevent that the vitamin K in the foodstuff is lost prematurely it is preferred to pack the

foodstuff in such a way that air and light are excluded and, optionally, to add an antioxidant innoxious for health, e.g. vitamin C (ascorbic acid) which is water-soluble, or vitamin E (tocopherol) which is fat-soluble.

Apart from vitamin K and fluorine compounds also other anti-caries agents can be incorporated in the foodstuff of the invention, such as :

- one or more nitrofurans, such as 2-nitro-5-furaldehydesemi-carbazone ("Furacin") and 5-nitro-2-furaldehyde-2-hydroxy-ethylsemi-carbazone ("Furadroxyl"), preferably in an amount of from 10 to 30 mg per 100 g of dry matter ;
- one or more natural edible substances having bacteriostatic activity, such as enzymes ; vegetable extracts, e.g. camomile and eucalyptus extracts ; herbs, such as thyme, camomile, and eucalyptus ; and aromatic oils, such as eucalyptus oil, sassafras oil, peppermint oil, camomile oil or thyme oil ; the inclusion of thyme oil, preferably in an amount of from 0.1 to 100 mg per 100 g of dry matter, is particularly preferred.

A survey of the substances that can play a role in the caries problem is found in Shafer-Hine-Levy, Textbook of Oral Pathology, W.B. Saunders Company, Philadelphia, U.S.A.

The invention also relates to the preparation of the above foodstuff with anti-caries activity. Generally the process of the invention for preparing the foodstuff with anti-caries activity is characterized in that in a suitable stage of the processing or preparation of the foodstuff at least one vitamin K is added thereto, in an amount effective for the reduction of caries.

The preferred embodiment of the process of the invention is characterized in that at a suitable stage of the processing or preparation of the foodstuff, besides one or more fluorine compounds, which provide from 0.001 to 0.75 mg of available fluorine per 100 g of dry matter, also at least one vitamin K is added, in an amount effective for the reduction of caries.

A further preferred embodiment of the process of the invention is characterized in that at a suitable stage at the processing or preparation of the foodstuff besides one or more fluorine compounds, which provide from 0.001 to 0.75 mg of available fluorine per 100 g dry matter, also at least one vitamin K is added, together with one or more iron compounds in an amount effective for the reduction of caries.

Preferably vitamin $K_1$ is included.

As mentioned before the invention especially relates to processes for preparing foodstuff wherein milk, or a product derived from milk or from one or more milk constituents, is the basis.
In a preferred embodiment the process for preparing a foodstuff relates to a foodstuff solid at body-temperature from milk or from one or more milk constituents, wherein at a suitable stage of the preparation the vitamin K and the iron compound and optionally the fluorine compound is added and uniformly blended.

According to a preferred embodiment cheese is prepared wherein vitamin K, iron and fluorine compound is added the vitamin K and optionally the fluorine compound are preferably added to the milk to be curded simultaneously with the rennet. As an alternative the vitamin K, iron compound and the fluorine compound can be added to the curd.

The fluorine compound can be added as a solution, or as a solid, optionally hydrated.

The vitamin K can be added as it is, or as a solution, suspension or emulsion in water or oil, or in any other form in which the vitamin K is available.

The invention relates to chewing-gum with anti-caries activity too.

With respect to a chewing-gum with anti-caries activity in DE-AS-1.956.016 a chewing-gum is mentioned which contains sodium mono fluorophosphate and vitamin $K_3$, see page 8, example 8.

This known chewing-gum contains besides the conventional gum base and the former compounds, pantothenic acid and rutin.

Pantothenic acid and rutin are technically less available compounds.

The invention avoids the drawback from technically less available compounds by inserting iron compound in the mixing formula of chewing-gum.

The chewing-gum of the invention may be prepared in a way known per se.

Apart from the gum base, the vitamin K and the iron and fluorine compounds the chewing-gum of the invention may comprise e.g. a sweetening agent, such as sorbitol, and a flavouring agent.

The invention relates to foodstuff, including chewing-gum characterized by the fact that these contain fluorine compound in relative minor amount with respect to the vitamin K and iron compound.

These foodstuff and chewing-gums are usable for people who might not be exposed to high levels of fluorine compounds.

In case of foodstuff including chewing-gum with low fluorine levels, these are characterized in that these contain :

a) vitamin K in an amount effective for the reduction of caries ;

b) one or more iron compounds in an amount from 1 to 1000 mg per 100 g of dry matter ;

c) fluorine compound, which provides from 0.001 to 0.01 mg of available fluorine per 100 g of dry matter.

The foodstuff, including chewing-gum, with relative minor amounts of fluorine, can be prepared by adding during a suitable stage of production the necessary vitamin K and iron to foodstuff, or chewing-gum base, which contain 0.001 to 0.01 mg of available fluorine per 100 g of dry matter.

The invention is illustrated by the following example. The percentages mentioned in the example are by weight, unless otherwise indicated.

Example 1 Baby-food

Into a double-walled, sterilized stainless steel container the following ,materials are introduced :
5088 kg of water (40 °C)
544 kg of milk (3.8% of fat)
146 kg of cream ( 40% of fat)
The mixture is heated to 50 °C, after which
40 kg of Ca-caseinate and
272 kg of malto-dextrin
are added. Subsequently the mixture is stirred for 10 min. Then 320 kg of whey powder are added, and the mixture is again stirred for 10 min. Then the following materials are added :
41 kg of whey powder
60 kg of glucose
2.1 kg of iron sulphate
1 g of NaF
After another 10 minutes period of stirring 83 kg of maize-oil and 83 kg of sunflower-oil are added. The resulting mixture is heated to 70 °C and homogenized in two stages (at 20.6 and 3.9 MPa, respectively). Then it is pasteurized at 90 °C for 3 sec., concentrated to a dry-matter content of 43% and dried in a spray-drying tower. About 1100 kg of pulverized baby-food are obtained. Finally 2 kg of minerals and vitamins, including 10 g of vitamin $K_1$, are added and thoroughly mixed.

**Claims**

**1.** A foodstuff containing vitamin K and iron and fluorine compounds for attaining anti-caries activity characterized in that :

a) it contains vitamin K in an amount effective for the reduction of caries ;

b) it contains one or more iron compounds in an amount from 1 to 1000 mg per 100 g of dry matter, assisting the caries resisting effect of the vitamin K ;

c) it contains fluorine compound, which provides from 0.001 to 0.75 mg of available fluorine per 100 g of dry matter ;

d) the iron and vitamin K are wholly or partly incorporated in a component of the foodstuff which component possesses the adherence to the teeth of cheese.

**2.** A foodstuff according to claim 1, characterized in that it is derived wholly or partly from milk.

**3.** A foodstuff according to claim 1 or 2 characterized in that the said vitamin K is vitamin K1.

**4.** A foodstuff according to any of the claims 1-3, characterized in that it comprises cheese as a basis.

**5.** A foodstuff according to claim 4 characterized in that the amount of fluorine compound, calculated as available fluorine, is from 0.1 to 0.4 mg per 100 g of dry matter.

**6.** A foodstuff according to claim 5 characterized in that the fluorine compound is sodium fluoride (NaF).

**7.** A foodstuff according to any one of the claims 1-6, characterized in that the fluorine compound is sodiumfluorophosphate ($Na_2PO_3F$).

**8.** A foodstuff according to any of the preceeding claims, characterized in that vitamin K has been included therein in an amount of from 0.001 to 100 mg per 100 g of dry matter.

**9.** A foodstuff according to any one of the foregoing claims, characterized in that one or more nitrofurans

has been included therein, in an amount of from 10 to 30 mg per 100 g of dry matter.

**10.** A foodstuff according to any one of the preceeding claims, characterized in that one or more natural edible substances with bacteriostatic action has been included therein.

**11.** A foodstuff according to claim 10, characterized in that thyme oil has been included therein, in an amount of from 0.1 to 100 mg per 100 g of dry matter.

**12.** A process for preparing a foodstuff with anti-caries activity according to claim 1, characterized that in a suitable stage of the processing or preparation of the foodstuff at least one vitamin K is added thereto in an amount from 1 to 1.000 mg per 100 g of dry matter.

**13.** A process for preparing a fluorine containing foodstuff with anti-caries activity according to claim 1, characterized in that besides one or more fluorine compounds, which provide from 0.001 to 0.75 mg of available fluorine per 100 g dry matter, also at least one vitamin K is added, together with one or more iron compounds in an amount effective for the reduction of caries.

**14.** A process according to claim 12 or 13, characterized in that the added vitamin K is vitamin K1.

**15.** A process according to any one of the claims 12 and 13, characterized in that a foodstuff having milk, or a product derived from milk or from one or more milk constituents as a basis, is prepared.

**16.** A process according to claim 15, characterized in that a foodstuff solid at body-temperature is prepared from milk or from one or more milk constituents and that in a suitable stage of the preparation the vitamin K and iron compound and optionally the fluorine compound is added and uniformly blended.

**17.** A process according to any one of the claims 13 to 15, characterized in that the added amount of fluorine compound, calculated as available fluorine, is from 0.1 to 0.4 mg per 100 g of dry matter.

**18.** A process according to any one of the claims 13 to 16, characterized in that sodium fluoride (NaF) is added.

**19.** Chewing-gum with anti-caries activity containing fluorine compounds and at least one vitamin K, characterized in that it contains an iron compound.

**20.** A process for preparing a chewing-gum with anti-caries activity, characterized in that a chewing-gum as defined in claim 19 is prepared in a way known per se.

**21.** Foodstuff including chewing-gum according to any of claims 1 and 19, characterized in that it contains
a) vitamin K in an amount effective for the reduction of caries ;
b) it contains one or more iron compounds in an amount from 1 to 1000 mg per 100 g of dry matter ;
c) it contains fluorine compound, which provides from 0.001 to 0.01 mg of available fluorine per 100 g of dry matter.

**22.** A process for preparing foodstuff including chewing-gum according to claim 21, characterized in that during a suitable stage of production vitamin K and at least one iron compound are added to foodstuff which contain 0.001 to 0.01 mg of available fluorine per 100 g of dry matter.

## Revendications

**1.** Produit alimentaire contenant de la vitamine K et des composés de fer et de fluor, pour obtenir une activité anti-caries, caractérisé en ce que :
a) le produit alimentaire contient de la vitamine K en quantité efficace pour réduire les caries ;
b) le produit alimentaire contient un ou plusieurs composés ferreux à raison de 1 à 1.000 mg pour 100 g de matière sèche, qui aide(nt) l'effet de résistance aux caries de la vitamine K ;
c) le produit alimentaire contient un composé fluoré qui apporte de 0,001 à 0,75 mg de fluor disponible pour 100 g de matière sèche;

d) le fer et la vitamine K sont complètement ou partiellement incorporés dans un composant du produit alimentaire, lequel composant possède l'adhérence du fromage sur les dents.

2. Produit alimentaire selon la revendication 1, caractérisé en ce qu'il est dérivé complètement ou partiellement du lait.

3. Produit alimentaire selon la revendication 1 ou 2, caractérisé en ce que ladite vitamine K est la vitamine $K_1$.

4. Produit alimentaire selon l'une quelconque des revendications 1 à 3, caractérisé en ce qu'il contient du fromage comme produit de base.

5. Produit alimentaire selon la revendication 4, caractérisé en ce que la quantité de composé fluoré, calculée sur la base de fluor disponible, est comprise entre 0,1 et 0,4 mg pour 100 mg de matière sèche.

6. Produit alimentaire selon la revendication 5, caractérisé en ce que le composé fluoré est le fluorure de sodium (NaF).

7. Produit alimentaire selon l'une quelconque des revendications 1 à 6, caractérisé en ce que le composé fluoré est le fluorophosphate de sodium ($Na_2PO_3F$).

8. Produit alimentaire selon l'une quelconque des revendications précédentes, caractérisé en ce qu'on y ajoute la vitamine K à raison de 0,001 à 100 mg pour 100 g de matière sèche.

9. Produit alimentaire selon l'une quelconque des revendications précédentes, caractérisé en ce qu'on peut y ajouter un ou plusieurs nitrofurannes, à raison de 10 à 30 mg pour 100 g de matière sèche.

10. Produit alimentaire selon l'une quelconque des revendications précédentes, caractérisé en ce qu'on y ajoute une ou plusieurs substances naturelles comestibles ayant une activité bactériostatique.

11. Produit alimentaire selon la revendication 10, caractérisé en ce qu'on y ajoute de l'essence de thym, à raison de 0,1 à 100 mg pour 100 g de matière sèche.

12. Procédé de préparation d'un produit alimentaire ayant une activité anti-caries selon la revendication 1, caractérisé en ce qu'à un stade approprié du traitement ou de la préparation du produit alimentaire, on ajoute à ce dernier au moins une vitamine K, à raison de 1 à 1.000 mg pour 100 g de matière sèche.

13. Procédé de préparation d'un produit alimentaire contenant du fluor et présentant une activité anti-caries selon la revendication 1, caractérisé en ce qu'on ajoute, en plus d'un ou plusieurs composés fluorés qui apporte(nt) 0,001 à 0,75 mg de fluor disponible pour 100 g de matière sèche, aussi au moins une vitamine K, conjointement avec un ou plusieurs composés de fer, en quantité efficace pour réduire les caries.

14. Procédé selon la revendication 12 ou 13, caractérisé en ce que la vitamine K ajoutée est la vitamine $K_1$.

15. Procédé selon l'une quelconque des revendications 12 et 13, caractérisé en ce qu'on prépare un produit alimentaire dont le produit de base est du lait ou un produit dérivé du lait ou d'un ou plusieurs constituants du lait.

16. Procédé selon la revendication 15, caractérisé en ce qu'on prépare un produit alimentaire solide à la température du corps, à partir du lait ou d'un ou plusieurs constituants du lait et qu'à un stade approprié de la préparation, on ajoute et mélange de façon homogène de la vitamine K et un composé de fer, et éventuellement le composé fluoré.

17. Procédé selon l'une quelconque des revendications 13 à 15, caractérisé en ce que la quantité ajoutée de composé fluoré, calculée en tant que fluor disponible, est comprise entre 0,1 et 0,4 mg pour 100 g de matière sèche.

18. Procédé selon l'une quelconque des revendications 13 à 16, caractérisé en ce qu'on ajoute du fluorure de sodium (NaF).

19. Gomme à mâcher ayant une activité anti-caries, contenant des composés fluorés et au moins une vitamine K, caractérisée en ce qu'elle contient un composé de fer.

20. Procédé de préparation d'une gomme à mâcher ayant une activité anti-caries, caractérisé en ce qu'on prépare, d'une manière connue en soi, une gomme à mâcher telle qu'elle est définie dans la revendication 19.

21. Produit alimentaire, y compris gomme à mâcher, selon l'une quelconque des revendications 1 et 19, caractérisé en ce qu'il contient :
   a) de la vitamine K en quantité efficace pour réduire les caries ;
   b) un ou plusieurs composés de fer à raison de 1 à 1.000 mg pour 100 g de matière sèche ;
   c) un composé fluoré qui apporte 0,001 à 0,01 mg de fluor disponible pour 100 g de matière sèche.

22. Procédé de préparation d'un produit alimentaire, y compris de la gomme à mâcher, selon la revendication 21, caractérisé en ce qu'à un stade approprié de la préparation, on ajoute de la vitamine K et au moins un composé de fer au produit alimentaire qui contient 0,001 à 0,01 mg de fluor disponible pour 100 g de matière sèche.

**Patentansprüche**

1. Nahrungsmittel mit einem Gehalt an Vitamin K sowie Eisen- und Fluorverbindungen zur Erzielung einer Antikarieswirksamkeit, dadurch gekennzeichnet, daß
   a) es Vitamin K in einer zur Herabsetzung von Karies wirksamen Menge enthält;
   b) es eine oder mehrere Eisenverbindungen in einer Menge von 1 bis 1.000 mg pro 100 g Trockensubstanz zur Unterstützung der Antikarieswirkung des Vitamins K enthält;
   c) es eine Fluorverbindung enthält, welche 0,001 bis 0,75 mg verfügbares Fluor pro 100 g Trockensubstanz liefert;
   d) wobei das Eisen und das Vitamin K ganz oder teilweise in eine Komponente des Nahrungsmittels einverleibt sind, die das Haftvermögen an den Zähnen von Käse besitzt.

2. Nahrungsmittel gemäß Anspruch 1, dadurch gekennzeichnet, daß es sich ganz oder teilweise von Milch ableitet.

3. Nahrungsmittel gemäß Anspruch 1 oder 2, dadurch gekennzeichnet, daß das Vitamin K Vitamin K 1 ist.

4. Nahrungsmittel gemäß einem der Ansprüche 1 bis 3, dadurch gekennzeichnet, daß es als Basis Käse enthält.

5. Nahrungsmittel gemäß Anspruch 4, dadurch gekennzeichnet, daß die Menge an der Fluorverbindung, berechnet als verfügbares Fluor, 0,1 bis 0,4 mg pro 100 g Trockensubstanz beträgt.

6. Nahrungsmittel gemäß Anspruch 5, dadurch gekennzeichnet, daß die Fluorverbindung Natriumfluorid (NaF) ist.

7. Nahrungsmittel gemäß einem der Ansprüch 1 bis 6, dadurch gekennzeichnet, daß die Fluorverbindung Natriumfluorophosphat ($Na_2PO_3F$) ist.

8. Nahrungsmittel gemäß einem der vorhergehenden Ansprüche, dadurch gekennzeichnet, daß Vitamin K in einer Menge von 0,001 bis 100 mg pro 100 g Trockensubstanz einverleibt wurde.

9. Nahrungsmittel gemäß einem der vorhergehenden Ansprüche, dadurch gekennzeichnet, daß ein oder mehrere Nitrofurane in einer Menge von 10 bis 30 mg pro 100 g Trockensubstanz in es einverleibt wurden.

10. Nahrungsmittel gemäß einem der vorhergehenden Ansprüche, dadurch gekennzeichnet, daß eine oder

mehrere natürliche eßbare Substanzen mit bakteriologischer Wirkung in es einverleibt wurden.

**11.** Nahrungsmittel gemäß Anspruch 10, dadurch gekennzeichnet, daß Thymianöl in einer Menge von 0,1 bis 100 mg pro 100 g Trockensubstanz in es einverleibt wurden.

**12.** Verfahren zur Herstellung eines Nahrungsmittels mit Antikarieswirksamkeit gemäß Anspruch 1, dadurch gekennzeichnet, daß in einer geeigneten Verfahrens- oder Herstellungsstufe des Nahrungsmittels zumindest ein Vitamin K in einer Mange von 1 bis 1.000 mg pro 100 g Trockensubstanz hinzugesetzt wird.

**13.** Verfahren zur Herstellung eines fluorhaltigen Nahrungsmittels mit Antikarieswirksamkeit gemäß Anspruch 1, dadurch gekennzeichnet, daß, neben einer oder mehreren Fluorverbindungen, welche 0,001 bis 0,75 mg verfügbares Fluor pro 100 g Trockensubstanz liefern, auch zumindest ein Vitamin K zugegeben wird, zusammen mit einer oder mehreren Eisenverbindungen in einer für die Herabsetzung von Karies wirksamen Menge.

**14.** Verfahren gemäß Anspruch 12 oder 13, dadurch gekennzeichnet, daß das zugegebene Vitamin K Vitamin K 1 ist.

**15.** Verfahren gemäß einem der Ansprüche 12 und 13, dadurch gekennzeichnet, daß ein Nahrungsmittel hergestellt wird, welches Milch oder ein von Milch oder von einem oder mehreren Milchbestandteilen abgeleitetes Produkt als Basis enthält.

**16.** Verfahren gemäß Anspruch 15, dadurch gekennzeichnet, daß ein bei Körpertemperatur festes Nahrungsmittel aus Milch oder einem oder mehreren Milchbestandteilen hergestellt wird und daß in einer geeigneten Verfahrensstufe das Vitamin K und die Eisenverbindung und ggf. die Fluorverbindung zugegeben und gleichförmig eingemischt werden.

**17.** Verfahren gemäß einem der Ansprüche 13 bis 15, dadurch gekennzeichnet, daß die zugegebene Menge an Fluorverbindung, berechnet als verfügbares Fluor, 0,1 bis 0,4 mg pro 100 g Trockensubstanz beträgt.

**18.** Verfahren gemäß einem der Ansprüche 13 bis 16, dadurch gekennzeichnet, daß Natriumfluorid (NaF) zugegeben wird.

**19.** Kaugummi mit Antikarieswirksamkeit und einem Gehalt an Fluorverbindungen und mindestens einem Vitamin K, dadurch gekennzeichnet, daß es zusätzlich eine Eisenverbindung enthält.

**20.** Verfahren zur Herstellung eines Kaugummis mit Antikarieswirksamkeit, dadurch gekennzeichnet, daß der in Anspruch 19 definierte Kaugummi in an sich bekannter Weise hergestellt wird.

**21.** Nahrungsmittel einschließlich Kaugummi gemäß einem der Ansprüche 1 und 19, dadurch gekennzeichnet, daß es enthält

a) Vitamin K in einer zur Herabsetzung von Karies wirksamen Menge;
b) eine oder mehrere Eisenverbindungen einer Menge von 1 bis 1.000 mg pro 100 g Trockensubstanz;
c) eine Fluorverbindung, die 0,001 bis 0,01 mg verfügbares Fluor pro 100 g Trockensubstanz liefert.

**22.** Verfahren zur Herstellung eines Nahrungsmittels einschließlich Kaugummi gemäß Anspruch 21, dadurch gekennzeichnet, daß während einer geeigneten Herstellungsstufe Vitamin K und zumindest eine Eisenverbindung zum Nahrungsmittel zugegeben werden, das 0,001 bis 0,01 mg verfügbares Fluor pro 100 g Trockensubstanz enthält.